# EUROPÄISCHE PATENTSCHRIFT

(11) **EP 3 125 965 B1**
(45) Veröffentlichungstag und Bekanntmachung des Hinweises auf die Patenterteilung: **12.12.2018**
(21) Anmeldenummer: 15712921.4
(22) Anmeldetag: 31.03.2015
(51) Int. Cl.: A61M 1/00, A61L 2/18, B08B 9/00, F04B 23/02, A61L 2/10, A61L 2/24, F04B 37/14, F04B 41/02

(54) **VORRICHTUNG ZUM REINIGEN EINER MEDIZINISCHEN VAKUUMPUMPE**
DEVICE FOR CLEANING A MEDICAL VACUUM PUMP
DISPOSITIF DE NETTOYAGE D'UNE POMPE À VIDE MÉDICALE

(30) Priorität: 04.04.2014 EP 14163495
(43) Veröffentlichungstag der Anmeldung: 08.02.2017
(73) Patentinhaber: Medela Holding AG, 6340 Baar (CH)
(72) Erfinder: EHLERT, Hilmar, 6052 Hergiswil (CH)
(74) Vertreter: Rutz, Andrea
(86) Internationale Anmeldenummer: PCT/EP2015/056936
(87) Internationale Veröffentlichungsnummer: WO 2015/150345

(56) Entgegenhaltungen:
- WO-A1-00/41745
- WO-A1-96/09080
- DE-A1-102010 021 240
- US-A- 5 736 098
- US-B1- 6 652 495

## Beschreibung

### TECHNISCHES GEBIET

Die vorliegende Erfindung betrifft eine Vorrichtung zum Reinigen einer medizinischen Vakuumpumpe.

### STAND DER TECHNIK

Wieder verwendbare portable Drainagepumpen, z.B. für die Wunddrainage oder für die Thoraxdrainage, müssen nach Gebrauch gereinigt werden, bevor sie für einen nächsten Patienten verwendet werden dürfen. Üblicherweise wird das Gehäuse der Pumpe abgewischt und desinfiziert. Das Innere der Pumpe, insbesondere die innen liegenden Schläuche bzw. der gesamte Vakuumpfad, lässt sich nicht reinigen. Deshalb ist es üblich, den Innenraum der Pumpe und die inneren Schläuche mittels eines Bakterien- und Überlauffilters zu schützen. Dieser Schutz durch Filter genügt in der Praxis jedoch oft nicht. Kleine Partikel oder Elemente können nach wie vor in den Vakuumpfad gelangen. Zudem besteht bei exzessiver Reinigung der Aussenseite die Gefahr, dass Reinigungsflüssigkeit in das Innere des Geräts gelangt und die Pumpe funktionsunfähig macht.

WO 2004/098801 offenbart eine Vorrichtung zur Reinigung von Kathetern, bei welcher über eine Druckleitung Wasser in den Katheter gespült und über eine Saugpumpe wieder abgesaugt wird. Ventile regeln den Wasserdruck.

WO 2011/003419 beschreibt eine Spülvorrichtung für Endoskope, wobei mittels einer peristaltischen Pumpe eine Reinigungsflüssigkeit durch das Lumen des Endoskops gepumpt wird.

WO 96/09080 offenbart eine spezielle Verbindung der Schläuche eines Dialysegeräts für Reinigungszwecke.

Diese Vorrichtungen sind jedoch nicht für die Reinigung von medizinischen Drainagepumpen ausgebildet.

### DARSTELLUNG DER ERFINDUNG

Es ist deshalb eine Aufgabe der Erfindung, eine Vorrichtung zur Reinigung einer medizinischen Vakuumpumpe, insbesondere einer Vakuummembranpumpe, zu schaffen, welche auch die Reinigung der inneren Schläuche der Pumpe ermöglicht.

Diese Aufgabe löst eine Vorrichtung mit den Merkmalen des Patentanspruchs 1.

Die erfindungsgemässe Vorrichtung dient der Reinigung einer medizinischen Vakuumpumpe, welche einen Sauganschluss und eine Abluftöffnung aufweist, die im Innern der Vakuumpumpe über einen Pfad miteinander verbunden sind. Der Pfad ist vorzugsweise fluiddicht. Er ist vorzugsweise der Vakuumpfad der Vakuumpumpe.
Die erfindungsgemässe Vorrichtung weist auf
- eine mit mindestens einem Spülmittelbehälter verbindbare Spülmittelzuführungsleitung mit einem ersten Anschluss zur Verbindung mit dem Sauganschluss und
- eine Saugleitung mit einem zweiten Anschluss zur Verbindung mit der Abluftöffnung und mit einem Vakuumanschluss zur Verbindung mit einer Saugquelle.
Diese Vorrichtung ermöglicht, ein Spülmittel vom mindestens einen Spülmittelbehälter durch die Zuführungsleitung und über den ersten Anschluss in die medizinische Vakuumpumpe und von dort über den zweiten Anschluss durch die Saugleitung in einen Abfluss zu saugen.

Dank dieser Vorrichtung mit der der zu reinigenden Vakuumpumpe nachgeschalteten Saugquelle lassen sich auch die inneren Lumen einer medizinischen Saugpumpe reinigen. Der Vakuumpfad der Pumpe lässt sich so auf einfache Art und Weise mit einem Reinigungsfluid, insbesondere einer Flüssigkeit, spülen, desinfizieren und dekontaminieren. Es lassen sich verschiedene Arten von Spülmittel durch den

Vakuumpfad saugen. Spülmittel sind beispielsweise Seifenlösungen, Desinfektionslösungen, und Luft. Der Vakuumpfad lässt sich zudem zum Schluss durch Spülung mit Luft trocknen.

Für die Reinigung ist es nicht notwendig, das Gehäuse der Pumpe zu öffnen und das Pumpaggregat und die Schläuche freizulegen. Vielmehr kann das gesamte Gerät, d.h. im Wesentlichen so wie es auch vom Patienten benützt wird, in die Reinigungsvorrichtung gelegt und dort gereinigt werden. Es ist kein Fachpersonal notwendig, um diese Reinigung durchzuführen. Insbesondere muss die Pumpe nicht demontiert und anschliessend wieder zusammengesetzt werden.

Die medizinische Vakuumpumpe ist beispielsweise eine Drainagepumpe, beispielsweise für die Wunddrainage oder die Thoraxdrainage, oder eine Brustpumpe zum Abpumpen von menschlicher Muttermilch. Sie kann beispielsweise eine Kolbenpumpe oder eine Membranpumpe sein.

Der genannte Sauganschluss der medizinischen Vakuumpumpe ist vorzugsweise der zum Anlegen des Vakuums beim Patienten oder bei der Mutter verwendete Sauganschluss. Es kann zur Reinigung jedoch auch ein zweiter Sauganschluss an der medizinischen Vakuumpumpe vorgesehen sein, wobei in diesem Fall der zum Saugen oder Abpumpen verwendete Sauganschluss vor dem Reinigen vorzugsweise verschlossen wird.

Die genannte Abluftöffnung ist vorzugsweise der sogenannte Auspuff der medizinischen Vakuumpumpe, durch welchen die in der Pumpe beim Erzeugen des Unterdrucks verdrängte Luft nach aussen gelangt. Analog zum Sauganschluss kann auch hier eine für die Reinigung speziell verwendete zweite Abluftöffnung vorhanden sein. In diesem Fall wird auch die im Gebrauch verwendete Abluftöffnung vorzugsweise verschlossen.

Die Verbindung zwischen der Reinigungsvorrichtung und der zu reinigenden medizinischen Vakuumpumpe ist vereinfacht, wenn der erste und zweite Anschluss der Reinigungsvorrichtung einfache Steckverbindungen sind. Die medizinische Vakuumpumpe lässt sich in diesem Fall einfach einstecken. Das Gerät wird manuell oder automatisch eingeschaltet und die Reinigung kann automatisch durchgeführt werden. In einer anderen Ausführungsform wird die Pumpe in die Vorrichtung eingelegt und die Steckverbindung erfolgt automatisch, indem die entsprechenden Anschlüsse der Vorrichtung motor- oder pneumatisch betrieben mit den entsprechenden Anschlüssen der Pumpe verbunden werden.

In einer Ausführungsform ist die Saugquelle Bestandteil der Vorrichtung, d.h. es ist eine Saugpumpe, vorzugsweise eine Kolbenpumpe oder eine Membranpumpe, vorhanden. In anderen Ausführungsformen weist die Vorrichtung einen Anschluss zur Verbindung mit einer externen Saugquelle, beispielsweise mit einem zentralen Spitalvakuumsystem, auf. Im zweiten Fall weist die Vorrichtung vorzugsweise ein Regulierventil auf, um das externe Vakuum zu regeln.

In einer einfachen Ausführungsform wird die zu reinigende medizinische Vakuumpumpe extern mit der Vorrichtung verbunden. Erfindungsgemäss weist die Vorrichtung aber eine Reinigungskammer auf, in welche die medizinische Vakuumpumpe eingelegt wird. Diese Kammer ist vorzugsweise mit einem Deckel verschliessbar, vorzugsweise ist sie flüssigkeits- und/oder gasdicht verschliessbar.

Vorzugsweise ist diese Reinigungskammer mit mindestens einem Mittel zur Reinigung einer äusseren Oberfläche der medizinischen Vakuumpumpe ausgestattet. Dieses Mittel ist beispielsweise mindestens eine UV-C-Lichtquelle und/oder eine Sprühvorrichtung und/oder eine Wischvorrichtung. Zudem kann eine Trocknungsvorrichtung zur Trocknung der äusseren Oberfläche vorhanden sein, beispielsweise ein Gebläse.

In einer Ausführungsform ist der mindestens eine Spülmittelbehälter ein beabstandet zur Vorrichtung angeordneter externer Spülmittelbehälter. In anderen Ausführungsformen ist mindestens einer der Spülmittelbehälter an oder in der erfindungsgemässen Vorrichtung in einer entsprechenden Aufnahme angeordnet. Vorzugsweise sind in dieser Variante alle Spülmittelbehälter an oder in der Vorrichtung gehalten.

Der mindestens eine Spülmittelbehälter kann in einfachen Ausführungsformen an der Aussenseite der Vorrichtung angeordnet sein oder er kann in der Reinigungskammer selber stehen. Vorzugsweise weist die Vorrichtung jedoch eine Gerätekammer für den mindestens einen Spülmittelbehälter auf, welche von der Reinigungskammer durch mindestens eine Trennwand getrennt ist. Vorzugsweise sind in diesem Fall erste und zweite Anschlüsse in der Trennwand angeordnet.

In einer Ausführungsform weist die Vorrichtung einen Fluidsammelbehälter auf, wobei der Abfluss in diesen Fluidsammelbehälter mündet. In einer anderen Ausführungsform führt der Abfluss nach aussen in das Abwassersystem des Spitals bzw. in einen externen Tank.

In einer Ausführungsform umfasst die Vorrichtung eine mit der Zuführleitung verbundene Ventileinheit zur gesteuerten Zuführung von Spülmittel aus dem mindestens einen Spülmittelbehälter und/oder von Luft in die Zuführungsleitung. Sind mehrere Spülmittelbehälter vorhanden, so ermöglicht die Ventileinheit ein Umschalten von einem ersten Spülmittel auf ein zweites Spülmittel oder auf Luft. Es ist auch ein Zuführen von Spülmitteln nacheinander möglich. Vorzugsweise ist die Ventileinheit mit einer elektronischen Steuereinheit der Vorrichtung verbunden, so dass ein automatisches Reinigungsverfahren durchgeführt werden kann, bei welchem in einem vorgegebenen Ablauf verschiedene Spülmittel und gegebenenfalls Luft in vorgegebenen Mengen und Zeitspannen durch die zu reinigende Pumpe gesaugt werden.

Vorzugsweise weist die Vorrichtung einen mit der Saugleitung verbundenen Fluiddetektions-Sensor auf. In einer Ausführungsform detektiert der Sensor, ob Spülmittel durch den gesamten Vakuumpfad gesaugt worden ist. Falls nicht, kann die Vorrichtung eine Fehlermeldung generieren oder je nach Ausführungsform eine automatische Fehlererkennung und wiederum je nach Ausführungsform eine Fehlerbehebung durchführen.

In einer einfachen Ausführungsform ist das Pumpaggregat der medizinischen Pumpe bei der Reinigung nicht aktiv, d.h. die Pumpe saugt nicht selber. In einer anderen Ausführungsform ist die Pumpe jedoch eingeschaltet und saugt das Reinigungsfluid durch den Vakuumkanal. Dies hat den Vorteil, dass die einzelnen Elemente der Pumpe in Bewegung sind und somit alle Bereiche gründlich gereinigt werden können.

Vorzugsweise weist die Vorrichtung Reinigungsprogramme auf, bei welchen die Saugquelle und gegebenenfalls auch die zu reinigende Pumpe lediglich zeitweise im Betrieb sind. Dadurch unterstützt die zu reinigende Pumpe zwar den Durchlauf des Spülmittels durch den Vakuumpfad, das Spülmittel kann jedoch auch einige Zeit unbewegt im Pfad verweilen und einwirken. In einer bevorzugten Variante wird das Spülmittel in einem ersten Schritt in den Vakuumpfad eingesaugt, bis der gesamte Pfad benetzt ist. Während einer Einwirkzeitspanne wird ohne Unterdruckapplikation gewartet und anschliessend wird das Spülmittel abgesaugt. Dieser Vorgang kann wiederholt oder es kann direkt Luft zum Trocken durchgesogen werden. Die Reinigung der äusseren Oberfläche durch Licht, Fluide oder andere Mittel kann vorher, gleichzeitig oder anschliessend erfolgen. Vorzugsweise erfolgt sie gleichzeitig, um die für die Reinigung benötigte Zeit zu minimieren.

In einer bevorzugten Ausführungsform weist die Vorrichtung eine Steuerungseinheit auf, mittels welcher die medizinische Vakuumpumpe betätigt werden kann und/oder mittels welcher in der medizinischen Vakuumpumpe gespeicherte Daten abgefragt und/oder mittels welcher Daten an die medizinische Vakuumpumpe übermittelt werden können. Vorzugsweise kann die Steuerungseinheit die Betriebsstunden der medizinischen Drainagepumpe feststellen und, falls eine manuelle zusätzliche Wartung notwendig ist, lässt sich diese Wartung anzeigen.

Wird die zu reinigende Pumpe durch die Vorrichtung gesteuert, so lässt sie sich vorzugsweise nicht nur ein- und ausschalten, sondern es lassen sich auch gezielt einzelne Parameter, wie Pumpfrequenz und maximaler Unterdruck, einstellen.

Die oben beschriebenen Ausführungsformen lassen sich miteinander kombinieren. Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung der erfindungsgemässen Vorrichtung zum Reinigen einer medizinischen Vakuumpumpe gemäss einer ersten Ausführungsform;
- Figur 2: eine schematische Darstellung der erfindungsgemässen Vorrichtung zum Reinigen einer medizinischen Vakuumpumpe gemäss einer zweiten Ausführungsform;
- Figur 3: eine schematische Darstellung der erfindungsgemässen Vorrichtung zum Reinigen einer medizinischen Vakuumpumpe gemäss einer dritten Ausführungsform und
- Figur 4: eine schematische Darstellung der erfindungsgemässen Vorrichtung zum Reinigen einer medizinischen Vakuumpumpe gemäss einer vierten Ausführungsform.

### BESCHREIBUNG BEVORZUGTER AUSFÜHRUNGSFORMEN

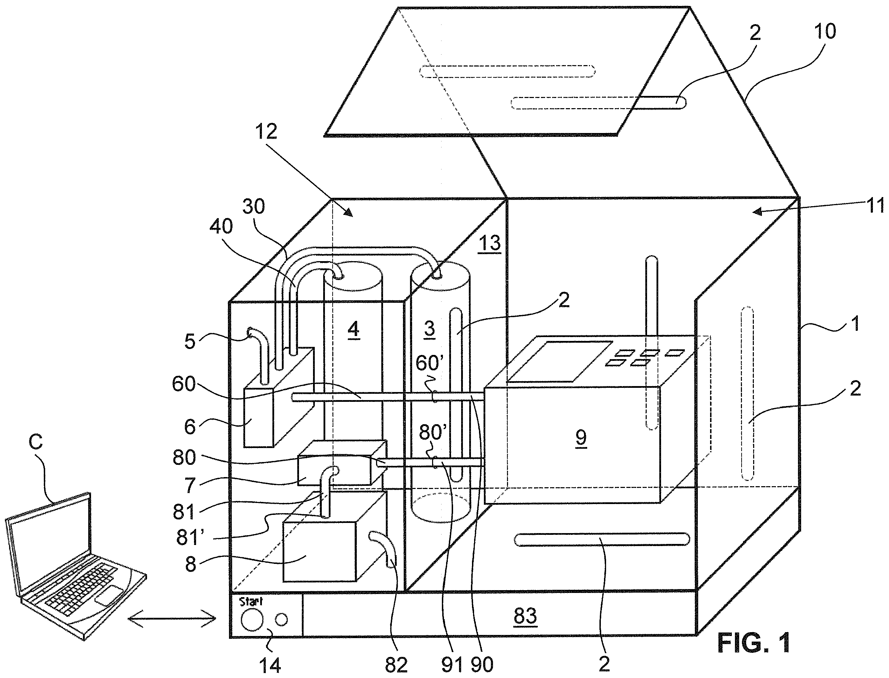

In Figur 1 ist ein erstes Ausführungsbeispiel einer erfindungsgemässen Vorrichtung zur Reinigung einer medizinischen Vakuumpumpe 9 dargestellt. Die Vorrichtung weist ein Gehäuse 1 auf. Im Gehäuse 1 sind zwei Kammern 11, 12 durch eine Trennwand 13 voneinander abgetrennt. Eine dieser Kammern ist eine Reinigungskammer 11, in welche die zu reinigende medizinische Vakuumpumpe 9 eingelegt werden kann. Eine andere Kammer ist als Gerätekammer 12 ausgebildet.

Ein schwenkbarer Deckel 10 ermöglicht einen Zugang zur Reinigungskammer 11 und verschliesst diese. Die Gerätekammer 12 ist vorzugsweise ebenfalls geschlossen. Sie kann ebenfalls einen den Zugang ermöglichenden Deckel aufweisen, wobei dieser Deckel hier nicht dargestellt ist.

Im Bodenbereich des Gehäuses 1 ist in diesem Beispiel ein Fluidsammelbehälter 83 angeordnet. Dieser ist vorzugsweise entfernbar im Gehäuse 1 gehalten, so dass er zwecks Entleerung der Vorrichtung entnommen werden kann.

Im Boden des Gehäuses 1 ist zudem eine elektronische Steuerungseinheit 14 angeordnet, wobei hier das Bedienpanel mit einem Start-Knopf abgebildet ist. Ein externer Computer C kann vorzugsweise über übliche drahtlose Verbindung und/oder Kabelverbindung mit dieser Steuereinheit 14 kommunizieren und Daten liefern sowie lesen.

Der Fluidsammelbehälter 83 sowie die Steuereinheit 14 können auch in anderen Bereichen des Gehäuses 1 angeordnet sein. Dasselbe gilt für die zwei Kammern 11, 12 und den Deckel 10. Auch in den weiter unten beschriebenen Ausführungsformen ist die dargestellte Anordnung der einzelnen Elemente lediglich beispielhaft und nicht einschränkend zu verstehen.

In der Gerätekammer 12 ist mindestens ein Spülmittelbehälter 3, 4 angeordnet. Hier sind zwei Spülmittelbehälter 3, 4 vorhanden. Diese Spülmittelbehälter 3, 4 können durch den Benützer befüllbar ausgebildet sein oder als geschlossene Einwegkartuschen dem Benützer geliefert werden. Sie beinhalten ein Spülmittel, beispielsweise eine Seifenlösung oder eine Desinfektionslösung.

Von jedem Spülmittelbehälter 3, 4 führt eine Spülmittelleitung 30, 40 zu einem Ventil einer Ventileinheit 6. Diese Ventileinheit 6 weist vorzugsweise zudem einen Lufteinlass 5 auf, welcher ebenfalls mit einem Ventil versehen ist. Der Lufteinlass 5 führt in diesem Beispiel in die Umgebung. Es ist jedoch auch möglich, dass der Lufteinlass 5 zu einem weiteren Behälter mit Luft, insbesondere mit Reinluft führt.

Die Ventileinheit 6 ist vorzugsweise mit der Steuereinheit 14 verbunden, welche die einzelnen Ventile der Einheit 6 in vorbestimmter Reihenfolge und Abfolge öffnet und schliesst und somit die einzelnen Spülmittel bzw. die Luft einer Zuführleitung 60 zuführt. Diese Verbindung ist in der Figur nicht dargestellt.

Die Zuführleitung 60 führt durch die Trennwand 13 in die Reinigungskammer 11 und endet in einem ersten Anschluss 60'. Eine erste Saugleitung 80 ragt mit einem ersten Ende ebenfalls durch die Trennwand 13 und endet dort in einem zweiten Anschluss 80'. Mit ihrem zweiten Ende führt sie zu einem Fluiddetektions-Sensor 7.

Eine zweite Saugleitung 81 führt vom Sensor 7 zu einer Saugpumpe 8. Sie bildet einen Vakuumanschluss 81'. Die erste und zweite Saugleitung 80, 81 bilden eine gemeinsame Saugleitung mit dem zweiten Anschluss 80' und dem Vakuumanschluss 81'.

Die Saugpumpe 8 ist vorzugsweise eine Membranpumpe. Sie kann jedoch auch eine Kolbenpumpe oder eine andere Art Pumpe sein. Sie kann lediglich als Aggregat ausgebildet sein oder sie kann im Gehäuse 1 von einem eigenen Gehäuse umgeben sein.

Eine Abflussleitung 82 führt von der Saugpumpe in den Fluidsammelbehälter 83. Dieser Behälter kann auch Teil der Pumpe 8 sein, so dass keine weitere Saugleitung notwendig ist. Insbesondere kann der Fluidsammelbehälter auch am Vakuumanschluss 81' angeordnet sein und der zum Absaugen des Spülmittels notwendige Unterdruck kann von der Pumpe 8 im Fluidsammelbehälter erzeugt werden.

Wie in Figur 1 dargestellt ist, ist die zu reinigende medizinische Vakuumpumpe 9 als Gesamtes, d.h. so wie sie vom Benützer verwendet wird, in die Reinigungskammer 11 eingelegt. Sie weist einen Sauganschluss 90 auf, an welchen üblicherweise der über einen Sammelbehälter zum Patienten oder direkt zur Mutter führende Vakuumschlauch angeschlossen wird. Ferner verfügt sie über eine Abluftöffnung 91, durch welche der bei Gebrauch der Pumpe 9 erzeugte Unterdruck aus dem Pumpaggregat entweicht. Zwischen diesen zwei Öffnungen 90, 91 erstreckt sich der Vakuumpfad der medizinischen Vakuumpumpe 9, inklusive Pumpkammer und allfälligen anderen zur Erzeugung des Vakuums notwendigen Bereiche. Er ist üblicherweise fluiddicht.

Der Sauganschluss 90 und die Abluftöffnung 91 sind hier schematisch dargestellt. Sie können beispielsweise einfache Öffnungen sein oder sie können mit einem Stutzen versehen sein. Es ist auch möglich, dass zur Reinigungsvorrichtung zugehörige mobile Stutzen in die als einfache Öffnungen ausgebildeten Anschlüsse 90, 91 eingesteckt werden.

Die Anschlüsse 90, 91 sind mit dem ersten und zweiten Anschluss 60', 80' verbunden, vorzugsweise durch eine Steckverbindung. Dadurch ist der Vakuumpfad der zu reinigenden Pumpe 9 in den Reinigungspfad der Reinigungsvorrichtung geschaltet. Es ist eine fluiddichte Verbindung von den Spülmittelbehältern 3, 4, über die Ventileinheit 6 und die Zuführungsleitung 60 zur Saugleitung 80, 81, dem Sensor 7 und der Saugpumpe 8 in den Fluidsammelbehälter 83 geschaffen. Der Vakuumpfad der zu reinigenden Pumpe 9 lässt sich somit spülen.

Zur Reinigung der Aussenseite der medizinischen Vakuumpumpe 9 verfügt die Reinigungskammer 11 vorzugsweise über entsprechende Mittel. In diesem Beispiel sind dies mehrere Lichtquellen, vorzugsweise UV-C-Lampen 2, welche verteilt an den Wänden der Reinigungskammer 11, vorzugsweise auch auf der Innenseite des Deckels 10, angeordnet sind und so die zu reinigende Pumpe 9 möglichst von allen Seiten bestrahlen. Alternativ oder zusätzlich können auch weitere Mittel, wie beispielsweise eine Sprühvorrichtung und/oder eine Wischvorrichtung, vorhanden sein. Deren Position kann in der Figur als ebenfalls mit der Bezugsziffer 2 versehen dargestellt sein.

Wie bereits im allgemeinen Teil dieser Beschreibung dargelegt, kann die Steuereinheit 14 vorzugsweise die zu reinigende Pumpe 9 während des Reinigungsvorgangs ein- und ausschalten und steuern und gegebenenfalls auch Daten auslesen bzw. neue Daten in die Pumpe 9 speichern.

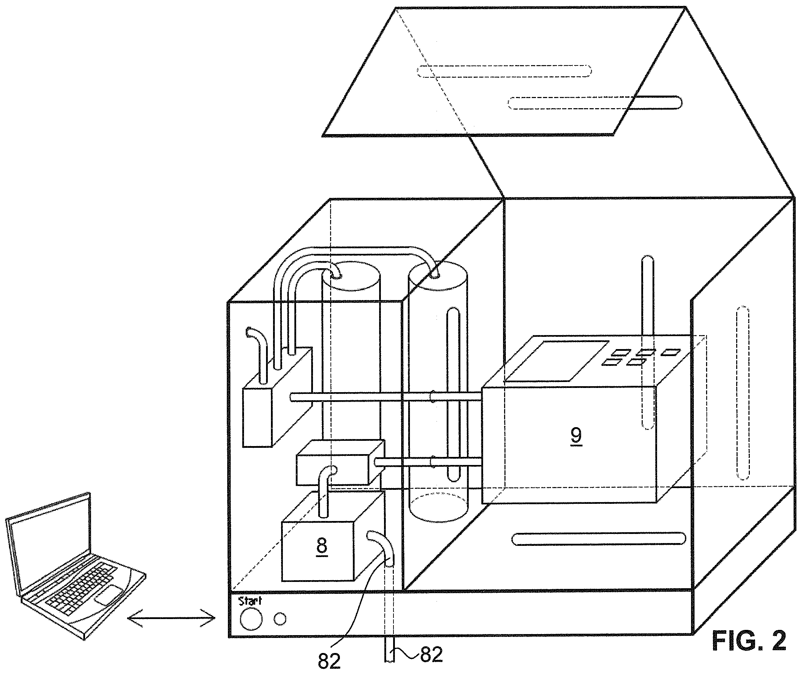

Nachfolgend sind anhand der Figuren 2 bis 4 weitere Ausführungsformen beschrieben. Es sind zur Erleichterung der Lesbarkeit der Figuren nicht mehr alle Bezugszeichen wiederholt, wobei gleich oder ähnlich dargestellte Teile nach wie vor gleich zu benennen und mit Bezugszeichen zu versehen sind wie im Ausführungsbeispiel gemäss Figur 1.

Im Beispiel gemäss Figur 2 ist kein Fluidsammelbehälter 83 vorhanden, sondern die Abflussleitung 82 führt aus dem Gehäuse 1 heraus in ein externes Abflusssystem oder in einen externen Tank.

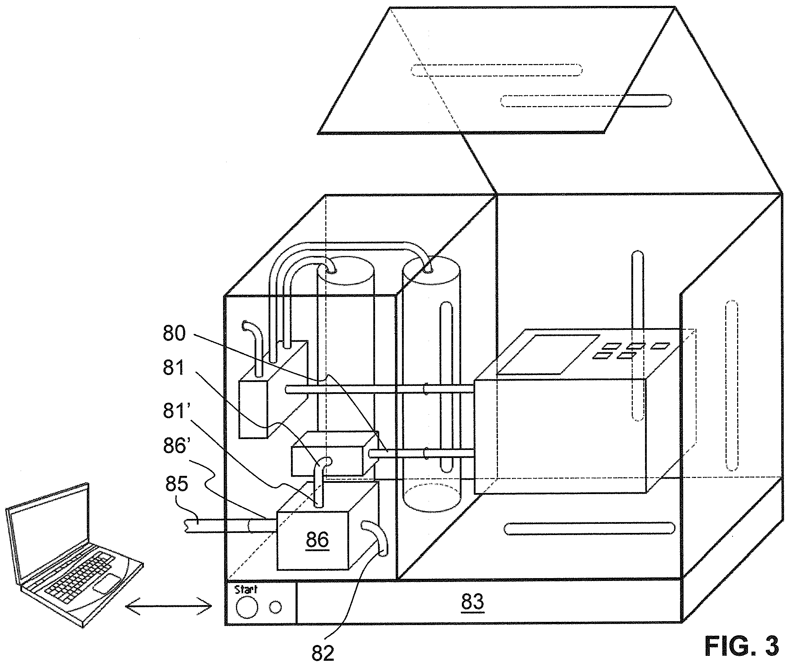

Im Beispiel gemäss Figur 3 ist wiederum ein Fluidsammelbehälter 83 vorhanden. Hier weist die Vorrichtung jedoch keine eigene Saugpumpe 8 auf. Es ist vielmehr ein Regulierventil 86 vorhanden, welches einerseits mit dem Vakuumanschluss 81' der Saugleitung 80, 81 und andererseits über einen Anschluss für eine externe Saugquelle 86' mit einer externen Saugquelle 85, beispielsweise einem zentralen Spitalvakuumsystem, verbindbar ist. Das Regulierventil 86 ist mit der Steuereinheit 14 verbunden und reguliert die Absaugung.

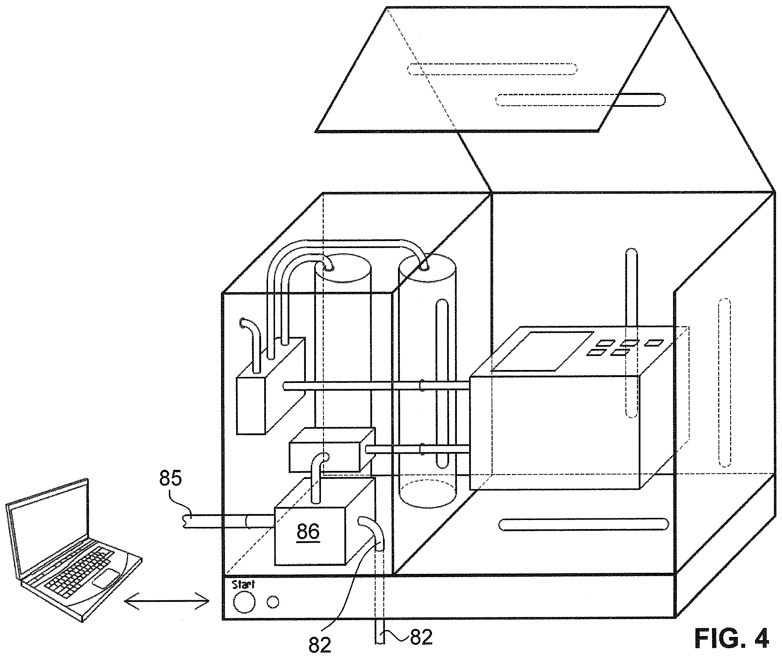

Im Beispiel gemäss Figur 4 führt die Abflussleitung 82 nach aussen und es wird eine externe Vakuumquelle verwendet.

Die erfindungsgemässe Vorrichtung ermöglicht eine einfache und schnelle Reinigung des Vakuumpfads von medizinischen Vakuumpumpen.

**BEZUGSZEICHENLISTE**

| | | | |
|---|---|---|---|
| 1 | Gehäuse | | |
| 10 | Deckel | 7 | Sensor |
| 11 | Reinigungskammer | | |
| 12 | Gerätekammer | 8 | Saugpumpe |
| 13 | Trennwand | 80 | erste Saugleitung |
| 14 | Steuerungseinheit | 80' | zweiter Anschluss |
| | | 81 | zweite Saugleitung |
| 2 | UV-C Lichtquelle | 81' | Vakuumanschluss |
| | | 82 | Abflussleitung |
| 3 | erster Spülmittelbehälter | 83 | Fluidsammelbehälter |
| 30 | erste Spülmittelleitung | 85 | externer Sauganschluss |
| | | 86 | Regulierventil |
| 4 | zweiter Spülmittelbehälter | 86' | Anschluss für externe Saugquelle |
| 40 | zweite Spülmittelleitung | | |
| | | 9 | medizinische Vakuumpumpe |
| 5 | Lufteinlass | 90 | Sauganschluss |
| | | 91 | Abluftöffnung |
| 6 | Ventileinheit | | |
| 60 | Zuführleitung | C | Computer |
| 60' | erster Anschluss | | |

## Patentansprüche

1. Vorrichtung zur Reinigung einer medizinischen Vakuumpumpe (9), wobei die medizinische Vakuumpumpe einen Sauganschluss (90) und eine Abluftöffnung (91) aufweist, welche im Innern der Vakuumpumpe (9) über einen Pfad miteinander verbunden sind, wobei die Vorrichtung aufweist:
- eine Reinigungskammer (11) zur Aufnahme der medizinischen Vakuumpumpe (9)
- eine mit mindestens einem Spülmittelbehälter (3, 4) verbindbare Spülmittelzuführungsleitung (60) mit einem ersten Anschluss (60') zur Verbindung mit dem Sauganschluss (90) und
- eine Saugleitung (80, 81) mit einem zweiten Anschluss (80') zur Verbindung mit der Abluftöffnung (91) und mit einem Vakuumanschluss (81', 86') zur Verbindung mit einer Saugquelle (8, 85), wobei ein Spülmittel vom mindestens einen Spülmittelbehälter (3, 4) durch die Spülmittelzuführungsleitung (60) und über den ersten Anschluss (60') in die medizinische Vakuumpumpe (9) und von dort über den zweiten Anschluss (80') durch die Saugleitung (80, 81) in einen Abfluss (82) saugbar ist.

2. Vorrichtung nach Anspruch 1, wobei der erste und der zweite Anschluss (60', 80') Steckverbindungen sind.

3. Vorrichtung nach einem der Ansprüche 1 oder 2, wobei sie ferner eine Saugpumpe (8) umfasst, welche mit dem Vakuumanschluss (81') der Saugleitung (80, 81) verbunden ist.

4. Vorrichtung nach einem der Ansprüche 1 oder 2, wobei sie ferner ein Regulierventil (86) aufweist, welches mit dem Vakuumanschluss (81') verbunden ist und welches einen Anschluss (86') zur Verbindung mit einer externen Saugquelle aufweist.

5. Vorrichtung nach einem der Ansprüche 1 bis 4, wobei die Reinigungskammer (11) mit mindestens einem Mittel (2) zur Reinigung einer äusseren Oberfläche der medizinischen Vakuumpumpe (9) ausgestattet ist.

6. Vorrichtung nach Anspruch 5, wobei das mindestens eine Mittel zur Reinigung mindestens eine UV-C-Lichtquelle (2) umfasst.

7. Vorrichtung nach einem der Ansprüche 5 oder 6, wobei das mindestens eine Mittel zur Reinigung eine Sprühvorrichtung (2) und/oder eine Wischvorrichtung umfasst.

8. Vorrichtung nach einem der Ansprüche 1 bis 7, wobei die Vorrichtung eine Aufnahme zur Aufnahme des mindestens einen Spülmittelbehälters (3, 4) aufweist.

9. Vorrichtung nach einem der Ansprüche 1 bis 7, wobei die Vorrichtung eine Gerätekammer (12) aufweist, welche von der Reinigungskammer (11) durch mindestens eine Trennwand (13) getrennt ist und wobei die Gerätekammer (12) eine Aufnahme zur Aufnahme des mindestens einen Spülmittelbehälters (3, 4) aufweist.

10. Vorrichtung nach Anspruch 9, wobei der erste und zweite Anschluss (60', 80') in der Trennwand (13) angeordnet sind.

11. Vorrichtung nach einem der Ansprüche 1 bis 10, wobei die Vorrichtung einen Fluidsammelbehälter (83) aufweist und wobei der Abfluss (82) in diesem Fluidsammelbehälter (83) mündet.

12. Vorrichtung nach einem der Ansprüche 1 bis 11, wobei die Vorrichtung eine mit der Spülmittelzuführungsleitung (60) verbundene Ventileinheit (6) umfasst zur gesteuerten Zuführung von Spülmittel aus dem mindestens einen Spülmittelbehälter (3, 4) und/oder von Luft in die Spülmittelzuführungsleitung (60).

13. Vorrichtung nach einem der Ansprüche 1 bis 12, wobei die Vorrichtung einen mit der Saugleitung (80, 81) verbundenen Fluiddetektions-Sensor (7) aufweist.

14. Vorrichtung nach einem der Ansprüche 1 bis 13, wobei sie eine Steuerungseinheit (14) aufweist zur Betätigung der medizinischen Vakuumpumpe (9) und/oder zur Abfrage von in der medizinischen Vakuumpumpe (9) gespeicherten Daten und/oder zur Übermittlung von Daten an die medizinische Vakuumpumpe (9).

## Claims

1. Device for cleaning a medical vacuum pump (9), wherein the medical vacuum pump has a suction connector (90) and an air removal opening (91), which are connected to each other in the interior of the vacuum pump (9) via a path, wherein the device has:
- a cleaning chamber (11) for receiving the medical vacuum pump (9)
- a rinsing agent delivery line (60), connectable to at least one rinsing agent container (3, 4) and having a first port (60') for connection to the suction connector (90), and
- a suction line (80, 81) having a second port (80') for connection to the air removal opening (91) and having a vacuum port (81', 86') for connection to a suction source (8, 85), wherein a rinsing agent can be sucked from the at least one rinsing agent container (3, 4) through the rinsing agent delivery line (60) and via the first port (60') into the medical vacuum pump (9) and from there via the second port (80') through the suction line (80, 81) into an outflow (82).

2. Device according to Claim 1, wherein the first and second ports (60', 80') are plug connections.

3. Device according to either of Claims 1 and 2, wherein it furthermore comprises a suction pump (8), which is connected to the vacuum port (81') of the suction line (80, 81).

4. Device according to either of Claims 1 and 2, wherein it furthermore has a regulator valve (86), which is connected to the vacuum port (81') and which has a port (86') for connection to an external suction source.

5. Device according to one of Claims 1 to 4, wherein the cleaning chamber (11) is equipped with at least one device (2) for cleaning an outer surface of the medical vacuum pump (9).

6. Device according to Claim 5, wherein the at least one device for cleaning comprises at least one UV-C light source (2).

7. Device according to either of Claims 5 and 6, wherein the at least one device for cleaning is a spraying device (2) and/or a wiping device.

8. Device according to one of Claims 1 to 7, wherein the device has a seat for receiving the at least one rinsing agent container (3, 4).

9. Device according to one of Claims 1 to 7, wherein the device has an equipment chamber (12), which is separated from the cleaning chamber (11) by at least one partition wall (13), and wherein the equipment chamber (12) has a seat for receiving the at least one rinsing agent container (3, 4).

10. Device according to Claim 9, wherein the first and second ports (60' 80') are arranged in the partition wall (13).

11. Device according to one of Claims 1 to 10, wherein the device has a fluid collection container (83), and wherein the outflow (82) opens out in this fluid collection container (83).

12. Device according to one of Claims 1 to 11, wherein the device comprises a valve unit (6) connected to the rinsing agent delivery line (60) for controlled delivery of rinsing agent from the at least one rinsing agent container (3, 4) and/or of air into the rinsing agent delivery line (60).

13. Device according to one of Claims 1 to 12, wherein the device has a fluid detection sensor (7) connected to the suction line (80, 81).

14. Device according to one of Claims 1 to 13, wherein it has a control unit (14) for actuating the medical vacuum pump (9) and/or for retrieving data stored in the medical vacuum pump (9) and/or for transmitting data to the medical vacuum pump (9).

## Revendications

1. Un dispositif de nettoyage d'une pompe à vide médicale (9), où la pompe à vide médicale comporte un raccord d'aspiration (90) et un orifice d'évacuation d'air (91), lesquels sont reliés l'un à l'autre à l'intérieur de la pompe à vide (9) à travers un circuit, où le dispositif comprend :
- une chambre de nettoyage (11) pour la réception de la pompe à vide médicale (9),
- une conduite d'alimentation de produit de rinçage (60), qui peut être reliée à au moins un réservoir de produit de rinçage (3, 4) équipée d'un premier raccord (60') pour une liaison au raccord d'aspiration (90) et
- une conduite d'aspiration (80, 81) équipée d'un deuxième raccord (80') pour une liaison à l'orifice d'évacuation d'air (91), et équipée d'un raccord de vide (81', 86') pour une liaison à une source d'aspiration (8, 85), où un produit de rinçage peut être aspiré de l'au moins un réservoir de produit de rinçage (3, 4) via la conduite d'alimentation de produit de rinçage (60) et à travers le premier raccord (60') dans la pompe à vide médicale (9) et de là, à travers le deuxième raccord (80') via la conduite d'aspiration (80, 81), dans un drain (82).

2. Dispositif selon la revendication 1, où le premier et le deuxième raccord (60', 80') sont des liaisons enfichables.

3. Dispositif selon une des revendications 1 ou 2, où celle-ci comprend en outre une pompe d'aspiration (8), laquelle est reliée au raccord de vide (81') de la conduite d'aspiration (80,81).

4. Dispositif selon une des revendications 1 ou 2, où en outre celle-ci comporte une soupape de réglage (86), laquelle est reliée au raccord de vide (81') et laquelle comporte un raccord (86') pour une liaison à une source d'aspiration externe.

5. Dispositif selon une des revendications 1 à 4, où la chambre de nettoyage (11) munie d'au moins un moyen (2) pour le nettoyage d'une surface extérieure de la pompe à vide médicale (9).

6. Dispositif selon la revendication 5, où l'au moins un moyen pour le nettoyage comprend au moins une source de lumière UV-C (2).

7. Dispositif selon une des revendications 5 ou 6, où l'au moins un moyen pour le nettoyage comprend un dispositif de pulvérisation (2) et/ou un dispositif d'essuyage.

8. Dispositif selon une des revendications 1 à 7, où le dispositif comporte une réception pour la réception d'au moins un réservoir de produit de rinçage (3, 4).

9. Dispositif selon une des revendications 1 à 7, où le dispositif comporte une chambre à équipement (12), laquelle est séparée de la chambre de nettoyage (11) par au moins une paroi de séparation (13) et où la chambre à équipement (12) comporte une réception pour la réception de l'au moins un réservoir de produit de rinçage (3, 4).

10. Dispositif selon la revendication 9, où le premier et le deuxième raccord (60', 80') sont disposés dans la paroi de séparation (13).

11. Dispositif selon une des revendications 1 à 10, où le dispositif comporte un réservoir de collecte de fluide (83) et où le drain (82) débouche dans ce réservoir de collecte de fluide (83).

12. Dispositif selon une des revendications 1 à 11, où le dispositif comprend une unité de soupape (6) reliée à conduite d'alimentation de produit de rinçage (60) pour une alimentation commandée en produit de rinçage provenant de l'au moins un réservoir de produit de rinçage (3, 4) et/ou en air dans la conduite d'alimentation de produit de rinçage (60).

13. Dispositif selon une des revendications 1 à 12, où le dispositif comporte un capteur de détection de fluide (7) relié à la conduite d'aspiration (80, 81).

14. Dispositif selon une des revendications 1 à 13, où celle-ci comporte une unité de commande (14) pour l'actionnement de la pompe à vide médicale (9) et/ou pour la consultation de données stockées dans la pompe à vide médicale (9) et /ou pour la transmission de données vers la pompe à vide médicale (9).
